(19)

**Europäisches Patentamt**
**European Patent Office**
**Office européen des brevets**

(11)  **EP 4 056 685 A1**

(12)  # EUROPEAN PATENT APPLICATION
published in accordance with Art. 153(4) EPC

(43) Date of publication:
**14.09.2022 Bulletin 2022/37**

(21) Application number: **19951810.1**

(22) Date of filing: **18.11.2019**

(51) International Patent Classification (IPC):
*C12N 5/095* (2010.01)          *C12N 5/00* (2006.01)
*C12N 5/071* (2010.01)

(52) Cooperative Patent Classification (CPC):
**C12N 5/00; C12Q 1/02**

(86) International application number:
**PCT/CN2019/119116**

(87) International publication number:
**WO 2021/088119 (14.05.2021 Gazette 2021/19)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.11.2019  CN 201911085968**

(71) Applicant: **Precedo Pharmaceuticals Co., Ltd.**
**Hefei, Anhui 230094 (CN)**

(72) Inventors:
 • **LIU, Qingsong**
  **Hefei, Anhui 230031 (CN)**
 • **LIU, Feiyang**
  **Hefei, Anhui 230031 (CN)**

 • **MEI, Husheng**
  **Hefei, Anhui 230031 (CN)**
 • **WANG, Wenchao**
  **Hefei, Anhui 230031 (CN)**
 • **ZHAO, Ming**
  **Hefei, Anhui 230031 (CN)**
 • **JIANG, Zongru**
  **Hefei, Anhui 230031 (CN)**
 • **REN, Tao**
  **Hefei, Anhui 230031 (CN)**
 • **WANG, Li**
  **Hefei, Anhui 230031 (CN)**

(74) Representative: **Abel & Imray LLP**
**Westpoint Building**
**James Street West**
**Bath BA1 2DA (GB)**

(54)  **PRIMARY BREAST EPITHELIAL CELL CULTURE MEDIUM, CULTURE METHOD, AND USE THEREOF**

(57)  Provided is a culture medium containing amphiregulin for culturing primary breast epithelial cells and a culture method involving using the medium. In the culturing method, primary cells are cultured on a culture vessel coated with an extracellular matrix gel by using the culture medium, and the primary cells grow on the culture vessel, which has been coated with the extracellular matrix gel, and proliferate rapidly under the combined action of nutrient factors and an extracellular matrix contained in the primary cell culture medium. The cell model obtained by the primary cell culture medium and the primary cell culture method can be used for the evaluating the efficacy of and screening drugs.

Figure 1

## Description

**Technical Field**

The invention relates to the technical field of medicine, in particular to a culture medium and a culture method for culturing or amplifying primary breast epithelial cells *in vitro,* and also to the use of the cultured cells in evaluating of drug efficacy and screening of drug.

**Background of the Invention**

Breast cancer is one of the main malignant tumors affecting women's health. The latest statistical results show that in the world, the incidence rate and the mortality rate of breast cancer ranks the first and the second in the incidence rate and the mortality rate of female malignancies, respectively. In recent years, although a lot of progress has been made in the research on the molecular typing and pathogenesis of breast cancer, the current standard drug treatment for breast cancer is still dominated by hormones and cytotoxic drugs, lacking individualized precision medication guidance. Because of the diversity and complexity of breast cancer classification and the high heterogeneity, it is difficult to substantially predict the efficacy of clinical drugs based on molecular or genetic diagnosis alone without functional testing (Adam A. Friedman et al., Nat Rev Cancer, 15(12): 747-56, 2015).

Functional testing refers to an *in vitro* method for detecting the sensitivity of antitumor drugs on cells of cancer patients. The key to apply this approach is to develop tumor cell models that have a short growth cycle and can represent the biological characteristics of breast cancer patients. In addition, the cell models should be easy to operate to quickly and efficiently predict the efficacy of clinical medication, so as to give cancer patients precise medication guidance in a timely manner. However, the normally low success rates of *in vitro* establishment of cell models from the primary tumor cells of cancer patients, the long growth cycles, and the problems such as excessive proliferation of mesenchymal cells (such as fibroblasts, and the like), all restrict the development in this field. There are currently two techniques for culturing primary epithelial/stem cells that are relatively mature in the field of tumor cell functional testing. One is the use of irradiated feeder cells and the ROCK kinase inhibitor Y27632 to promote the growth of primary epithelial cells to investigate the drug sensitivity on individual patients, that is, the cell conditional reprogramming technology (Liu et al., Am J Pathol, 180: 599-607, 2012). Another technique is 3D culture of adult stem cells *in vitro* to obtain organoids similar to tissues and organs (Hans Clevers et al., Cell, 11; 172(1-2): 373-386, 2018).

However, both techniques have certain limitations. Cell reprogramming is a technique in which a patient's autologous primary epithelial cells are co-cultured with murine-derived feeder cells; however, the presence of these murine-derived cells may interfere with the drug sensitivity test results of the patient's autologous primary cells during drug sensitivity testing on the patient's primary cells; on the other hand, if murine-derived feeder cells are knocked out, the patient's autologous primary cells may be removed from the reprogramming environment, and the cell proliferation rate and the intracellular signaling pathway may be significantly changed (Liu et al., Am J Pathol, 183(6): 1862-1870, 2013; Liu et al., Cell Death Dis., 9(7): 750, 2018), which leads to a result that the response of the patient's autologous primary cells to the drug is greatly affected. The organoid technology is a technology that embeds the patient's autologous primary epithelial cells into the extracellular matrix for 3D *in vitro* culture, which does not require feeder cells, and thus, there is no interference problem of murine-derived feeder cells. However, the medium of the organoid technology needs to be added with a variety of specific growth factors, which is expensive and unsuitable for extensive use in clinical. In addition, the organoid needs to be embedded in the extracellular matrix gel during the entire culture process, and the plating steps of cell inoculation, passage, and drug sensitivity test are cumbersome and time-consuming as compared to 2D culture operations. Additionally, the size of the organoid formed by this technology is difficult to control, and some organoids may grow too large and cause an internal necrosis. Therefore, the organoid technology has poorer operability and applicability than the 2D culture technology. It requires professional technicians to operate, and thus, is not suitable for extensive and wide use in clinical for *in vitro* drug sensitivity testing (Nick Barker, Nat Cell Biol, 18(3): 246-54, 2016).

In view of the limitations of the above technologies, it is necessary to develop a culture technology for primary breast epithelial cells in clinic, which may provide short culture period, controllable cost, convenient operation, without interference from exogenous cells. When the technology is applied to construct a primary breast tumor cell model, the cultured breast tumor cells can represent the biological characteristics of the breast cancer patients. By evaluating *in vitro* the sensitivity of anti-tumor drugs in the cell models derived from individual cancer patients, the response rate of the anti-tumor drug can be improved in clinic, and the pains caused by inappropriate drugs to the patients and the wastes of medical resources can be reduced.

**Summary of the Invention**

In view of the deficiencies of the prior art, the invention intends to provide a primary breast epithelial cell culture medium for culturing primary breast epithelial cells and a method for culturing primary breast epithelial cells using the culture medium. The primary breast epithelial cell culture medium and the culture method of the invention can achieve the goal of short *in vitro* culture period, controllable cost, convenient operation and no interference from exogenous cells. When the technology is applied to construct a primary breast tumor cell model, the primary breast tumor cells with the biological characteristics of the breast cancer patients can be obtained, which can be applied in new drug screening and *in vitro* drug sensitivity test.

One aspect of the invention is to provide a primary cell culture medium for culturing primary breast epithelial cells, comprising amphiregulin, wherein, the content of amphiregulin is 10 ng/ml or more, and from the viewpoint of cost, preferably 10 ng/ml to 100 ng/ml.

The primary cell culture medium of the invention preferably further comprises one or more or all of the following: epidermal growth factor (EGF), insulin, B27, ROCK kinase inhibitor Y27632, Neuregulin 1, fibroblast growth factor 7 (FGF7), TGFβ type I receptor inhibitor A8301, and P38/MAPK inhibitor SB202190. Preferably, the content of the EGF is 2.5 ng/ml-20 ng/ml; the content of the insulin is 1 μg/ml-10 μg/ml; the B27 is diluted with the final concentration of 1:25-1:100; the content of the Y27632 is 5 μM-15 μM; the content of the Neuregulin 1 is 5 nM-20 nM; the content of the FGF7 is 2.5 ng/ml-20 ng/ml; the content of the A8301 is 100 nM-500 nM; the content of the SB202190 is 100 nM-500 nM.

Compared to the cell conditional reprogramming medium and the breast epithelial cell organoid medium, the composition of this medium is supplemented with amphiregulin, but does not contain uncertain components such as serum, bovine pituitary extract or the like, niche factors that are necessary for organoid culture such as Wnt agonists, R-spondin family proteins, BMP inhibitors or the like, nor does it contain fibroblast growth factor 10 (FGF10), nicotinamide, and N-acetylcysteine, thereby greatly reducing the cost of the medium, simplifying the operation process of preparing the medium, and realizing the *in vitro* culture of the primary breast epithelial cells with controllable cost and convenient operation.

In the invention, the primary breast epithelial cells can be selected from breast tumor cells, normal breast epithelial cells, and breast epithelial stem cells.

One aspect of the invention is to provide a method for culturing the primary breast epithelial cells, comprising the following steps:

(1) Preparing the primary cell culture medium of the invention according to the above composition.
(2) Coating a culture vessel with extracellular matrix gel diluent.
Wherein, as the extracellular matrix gel, a low growth factor type extracellular matrix gel can be used, for example, the commercial Matrigel (Corning: 354230) or BME (Trevigen: 3533-010-02) can be used. More specifically, the extracellular matrix gel is diluted with a serum-free medium, which may be the primary cell medium of the invention or DMEM/F12 (Corning: R10-092-CV). The dilution ratio of the extracellular matrix gel is 1:50-400, preferably 1:100-200. The coating method comprises adding the diluted extracellular matrix gel into the culture vessel to cover the bottom of the culture vessel completely, and standing for 30 minutes or more, preferably standing and coating at 37°C for 30-60 minutes. After the coating is completed, the excess extracellular matrix gel diluent is discarded, and the culture vessel is ready for later use.
(3) Isolating the primary breast epithelial cells from the breast tissue.

The primary breast epithelial cells can be derived, for example, from the breast cancer tissue samples and paracancerous tissue samples. For example, the breast cancer tissue samples are derived from the cancer tissue of the informed and consenting breast tumor patient by surgical resection, and the paracancerous tissue samples are collected from the breast tissue at least 5 cm away from the breast cancer tissue. Collection of the aforementioned tissue samples is performed within half an hour of the surgical excision or the biopsy. More specifically, in a sterile environment, the tissue sample from non-necrotic sites is cut, with its volume of more than 0.5 $cm^3$, and then the tissue sample is placed in pre-cooled 10-50 mL DMEM/F12 medium, which is contained in a plastic sterile centrifuge tube with a lid, and transported to the laboratory on ice. Wherein, the DMEM/F12 medium contains 50-200 U/mL (e.g. 100 U/mL) penicillin and 50-200 U/mL (e.g. 100 U/mL) streptomycin (hereinafter referred to as the transport fluid).

In a biological safety cabinet, the tissue sample is transferred to a cell culture dish, which is then rinsed with the transport fluid, the blood cells on the surface of the tissue sample are washed away, and unnecessary tissues such as skin and fascia on the surface of the tissue sample are removed.

The rinsed tissue sample is transferred to another new culture dish, with the addition of 5-25 mL of the transport fluid, and the tissue sample is divided into tissue fragments less than 1 $mm^3$ in diameter using a sterile scalpel blade and a forceps.

The tissue sample fragments are transferred to a centrifuge tube, which is centrifuged at 1000 rpm or more for 3-10 minutes in a tabletop centrifuge; after carefully removing the supernatant from the centrifuge tube with a pipette, it is resuspended using 5-25 mL of serum-free DMEM/F12 medium containing collagenase II (0.5-5 mg/mL, e.g. 1 mg/mL) and collagenase IV (0.5-5 mg/mL, e.g. 1 mg/mL), and is shaken digested on a shaker with constant temperature of 37°C for at least 1 hour (digestion time depends on the sample size; if the sample is larger than 1 g, the digestion time is increased to 1.5-2 hours), then it is centrifuged at 300 g/min or more for 3-10 minutes in a tabletop centrifuge; after discarding the supernatant, the digested tissue cells is resuspended with 5-25 mL of DMEM/F12 medium containing, for example, 10% fetal bovine serum, and then is grinded and sieved, with the cell sieve aperture of, for example, 100 $\mu$m, and the sieved cell suspension is collected in centrifuge tubes; the cells are counted with a hemocytometer.

The cell suspension is then centrifuged at 300 g/min or more for 3-10 minutes in a centrifuge; after discarding the supernatant, it is resuspended in the primary cell culture medium of the invention.

(4) Inoculating the primary breast epithelial cells isolated in step (3) in the coated culture vessel.

More specifically, the primary breast tumor cells are inoculated at a density of $1 \times 10^3$ to $1 \times 10^5$ cells/mL (e.g., $1 \times 10^4$ cells/mL) in a T12.5 culture flasks; 1-10 mL of primary epithelial cell culture medium is added, and then is cultured in a cell incubator for 8-10 days, for example, under the conditions of 37°C, 5% $CO_2$, with either normoxic (15-20% oxygen concentration) or hypoxia (0.5-4 % oxygen concentration) oxygen concentration; fresh primary cell culture medium is used for replacing every 4 days during the culture; digestion and passaging are preformed when the primary breast epithelial cells grow to a cell density that accounts for about 80% to 90% of the bottom area of the culture flask.

This inoculation step does not require the use of feeder cells, and compared to the cell conditional reprogramming technology, the operation steps of culturing and irradiating feeder cells are omitted. Compared to the organoid technology, this step does not need to mix the primary cells with the matrix gel uniformly on ice to form gel droplets and wait for the solidification of the gel droplets before adding the medium. The pre-coated culture vessel can be used directly for inoculation of the primary cells. In addition, only a small amount of diluted extracellular matrix gel is needed to coat culture vessels, which results to the saving of expensive extracellular matrix gel and simplification of the operation steps as compared with the organoid technology.

(5) Optionally, after culturing the inoculated primary breast epithelial cells for 4-10 days, when the maximum diameter of the cell clones formed in the culture flask reaches 500 $\mu$m, the supernatant is discarded, and 1-2 mL of 0.05% trypsin (Thermo Fisher: 25300062) is added for cell digestion, which is then incubated at room temperature for 5-20 min. The digested cells are resuspended in 1-10 mL of medium containing, for example, 10% (v/v) fetal bovine serum, 100 U/mL penicillin, and 100 U/mL streptomycin, and are centrifuged at 300 g/min or more for 3-10 minutes. The digested single cells are resuspended using the primary cell culture medium of the invention, and the obtained cell suspension is placed in a T25 cell culture flask coated with extracellular matrix gel for continuous culture. The coating operation of T25 cell culture flask is the same as that in step (2).

The expanded breast epithelial cells grow in 2D, avoiding the nonuniform size of organoids and internal necrosis in overgrown organoids that may occur in the expansion using organoid technology.

In addition, the breast epithelial cells, especially the breast tumor cells, cultured by the culturing method for primary breast epithelial cells of the invention, can be used for drug efficacy evaluation and drug screening, which comprises the following steps:

(1) Obtaining the primary breast epithelial cells, more preferably, obtaining the cancer tissue samples or the biopsy cancer tissue samples derived from the breast cancer patients; isolating the primary breast epithelial cells, and culturing and expanding the primary breast epithelial cells (particularly the primary breast tumor cells) according to the method described above to a cell number of at least the magnitudes of $10^5$, preferably at least the magnitudes of $10^6$.

(2) Selecting the drug for testing.

(3) Based on the maximum plasma concentration of the drug Cmax as a reference, taking 2-5 times of Cmax as the initial concentration, and diluting the drug into different drug concentration gradients, such as 5-10 drug concentration gradients, preferably 6-8 drug concentration gradients.

(4) Digesting the breast epithelium cultured in step (1) into a single cell suspension, counting the cell number with a hemocytometer, diluting the single cell suspension with the primary cell culture medium of the invention containing extracellular matrix gel, adding the diluted cell suspension evenly to the multi-well plate at a density of 1000-10000 cells per well, e.g., 50 $\mu$L of cell dilution per well, and adhering overnight.

This step avoids the problem of the cell reprogramming technique that the presence of feeder cells may interfere with the primary cell counting and the subsequent primary cell viability assay, and eliminates the need of the tedious step of mixing, embedding and then plating the cell suspension with matrix gel on ice as that in the organoid technique, which greatly simplifies the operation process and enhances the operability and practicality of the technology. Since the inoculated cells are single-cell suspensions rather than 3D structures like organoid, this technique may result in a more uniform number of plating cells and less variation in cell numbers between wells when compared to the organoid technique, making it more suitable for subsequent high-throughput drug screening operations.

(5) Adding the selected candidate drugs such as traditional chemotherapeutic drugs, targeted drugs, antibody drugs, or combination thereof with gradient dilutions, to the adherent cells obtained in step (4), using a high-throughput automated workstation.

(6) After a few hours of drug addition, for example, 72 hours, detecting the survival rate of the breast epithelial cells using the Cell-Titer Glo luminescence cell viability detection kit (Promega: G7573) to screen for drug activity.

Specifically, each well is added with, for example, 50 μL of Cell Titer-Glo reagent (Promega: G7573), and after uniformly shaking, the chemiluminescence intensity of each well is measured with a fluorescence microplate reader. Taking the drug concentration as the abscissa and the fluorescence intensity as the ordinate, GraphPad Prism 7.0 software is used to draw the drug dose-effect curve based on the measured values, and the inhibitory intensity of the drugs on the proliferation of the tested cells are calculated.

When using the primary breast tumor cells of the invention in drug screening and *in vitro* drug sensitivity test, since it is not a cell co-culture system, the phenomenon that feeder cells in the cell reprogramming technology interfere with the test results will not occur. Due to the 2D growth of the cells, the interaction with the drug is also quicker than the drug test time in the organoid technology (the average administration time in the organoid technology is 6 days).

The beneficial effects of the invention also include:

(1) the success rate for culturing the primary breast epithelial cell can be improved, with a success rate of more than 90%;

(2) the breast epithelial cells primary cultured *in vitro* can be ensured to reproduce the pathological phenotype and the heterogeneity of the owner patient of the primary cells;

(3) the primary breast epithelial cells cultured are pure breast epithelial cells which are not interfered by mesenchymal cells such as fibroblasts and adipocytes ;

(4) the composition of the medium does not contain serum, and thus, it is not affected by the quality and the quantity of the serum from different batches;

(5) the breast epithelial cells can be expanded with high efficiency, with a magnitude of $10^6$ of breast epithelial cells being successfully expanded within about two weeks from the starting of $10^4$-level cell count, and the expanded breast epithelial cells have the ability of continuous passage;

(6) there is no need to operate on ice and to dissociate the matrix gel in the passage step, and the digestion and passage of cells can be completed within 10-15 minutes;

(7) the cost for culture is controllable, as the primary breast cancer medium does not require expensive Wnt agonists, R-spondin family proteins, BMP inhibitors, FGF10 and the like factors, and thus, it is a simplification and improvement of the existing organoid culture medium for primary breast epithelial cells; and the cell inoculation does not need to use a higher concentration of extracellular matrix for mixing with the primary cells to form gel droplets, but instead, only a small amount of extracellular matrix gel dilution is required, which saves the amount of costly extracellular matrix;

(8) the operations are convenient: compared with the conditional reprogramming technology, this technology does not need to cultivate or irradiate the feeder cells, which avoids the problem that the quality and the quantity of feeder cells from different batches may affect the efficiency of primary cell culture, and the plating and testing objects in the drug screen are only primary breast epithelial cells, without the interference of feeder cells in the co-culture system as described in the cell conditional reprogramming technology; compared with the organoid technology, in the method for coating extracellular matrix gel adopted in the invention, the culture vessel can be prepared in advance, and there is no need to embed cells in the matrix gel as in the organoid technology, and the operation steps are simple and easy;

(9) this technology can culture and provide the breast epithelial cells with large quantity and high uniformity, which is suitable for high-throughput screening of new candidate compounds and high-throughput drug sensitivity functional tests *in vitro* for patients.

Using the cell culture medium of this embodiment, the breast epithelial cells derived from humans or other mammals, including breast tumor cells, normal breast epithelial cells, breast epithelial stem cells, or tissues comprising at least any of these cells, can be cultured.

In addition, organoids can also be formed from at least one of the cells and the tissues.

Furthermore, the cells obtained by the culture method of this embodiment can be used in regenerative medicine, basic medical research of breast epithelial cells, screening of drug responses, and development of new drugs for breast diseases, and the like.

**Description of Drawings**

Figure 1 are photographs under an inverted phase contrast microscope of the primary breast epithelial cells obtained by culturing the cells, where are isolated from clinical breast tissue samples, using the primary cell culture method of the invention.

Figure 2 are photographs under microscope of the cells, which were obtained by inoculating and culturing the primary breast tumor cells isolated from two different breast cancer clinical tissue samples (HMFL-XN30, HMFL-XN22) in culture plates coated with extracellular matrix gel and without any coating.

Figure 3 are graphs for illustrating the effect of amphiregulin on the proliferation of primary breast tumor cells.

Figure 4 are the comparison of cell growth curves obtained by culturing the cells isolated from two breast cancer clinical tissue samples (HMFL-XN12, HMFL-XN21) using the cell conditional reprogramming technology, the technology of the invention and the organoid technology, respectively, and the photographs under microscope of HMFL-XN21 cultured to day 27.

Figure 5 are photographs under inverted microscope of the breast tumor cells, which were isolated from a breast cancer clinical tissue sample (HMFL-XN12) and cultured to day 9 and day 22 using the technology of the invention.

Figure 6 are the comparison of immunofluorescence staining results of the breast tumor cells, which were isolated from a surgically resected sample of breast cancer (HMFL-XN7) and cultured by the technology of the invention, with the immunohistochemical results of the original tissue section of the tissue sample.

Figure 7 shows the gene mutation consistency analysis of the breast tumor cells of different passages, which were obtained by culturing the cells isolated from two cases of breast cancer surgical resection samples using the technology of the invention, and the chromosome karyotype analysis result of one case.

Figure 8 shows the tumorigenicity of the breast tumor cells in mice obtained by culturing the primary breast tumor cells derived from the cancer tissues of two cases of pathologically diagnosed triple-negative breast cancer patients using the technology of the invention.

Figure 9 are graphs showing the dose-response curves of the primary breast tumor cells cultured by the technology of the invention on different chemotherapeutic drugs and targeted drugs, and the calculated half-inhibition rate.

**Detailed Description of the Invention**

[Example 1]

Isolation of human primary breast epithelial cells and optimization of primary breast epithelial cell culture medium

(1) Isolation of human primary breast epithelial cells

Breast cancer tissue samples were obtained from the cancer tissues of five informed and consenting breast tumor patients by surgical resection, namely HMFL-XN1, HMFL-XN3, HMFL-XN4, HMFL-XN6, and HMFL-XN8. One of the samples (HMFL-XN1) will be described below. The aforementioned tissue samples were collected within half an hour after the surgical excision or the biopsy. More specifically, in a sterile environment, tissue samples from non-necrotic sites were cut with a volume of more than 0.5 $cm^3$ and were placed in 20 mL of pre-cooled DMEM/F12 medium (manufactured by Corning Inc.). The medium was placed in a 50 mL plastic sterile centrifuge tube with a lid and transported to the laboratory on ice; wherein, the DMEM/F12 medium contained 100 U/mL penicillin and 100 U/mL streptomycin (hereinafter referred to as the transport fluid).

In the biological safety cabinet, the tissue sample (HMFL-XN1) was transferred to a 100 mm cell culture dish. The tissue sample was rinsed with the transport fluid. The blood cells on the surface of the tissue sample was washed away. Unwanted tissues such as skin and fascia on the surface of the tissue sample were removed.

The rinsed tissue sample was transferred to another new 100 mm culture dish; 10 mL of transport fluid was added, and a sterile scalpel blade and a forceps were used to divide the tissue sample into tissue fragments less than 1 $mm^3$ in diameter.

The tissue sample fragments were transferred to a 50 mL centrifuge tube, and centrifuged at 1200 rpm for 5 minutes using a table-top centrifuge; after carefully removing the supernatant from the centrifuge tube with a pipette, the rest was resuspended in 10 mL serum-free DMEM/F12 medium containing collagenase II (1 mg/mL) and collagenase IV (1 mg/mL). It was placed on a shaker with constant temperature of 37°C for shaking digestion for 1 hour and then was centrifuged at 350 g/min in a tabletop centrifuge for 5 minutes. After discarding the supernatant, the digested tissue cells were resuspended in 10 mL of DMEM/F12 medium containing 10% fetal bovine serum, and then it was grounded and sieved, with the cell sieve aperture of 100 μm, and the sieved cell suspension was collected in a 50 mL centrifuge tube; the cells were counted with a hemocytometer.

The cell suspension was then centrifuged at 350 g/min for 5 minutes in a centrifuge; after discarding the supernatant, it was resuspended in the primary cell culture medium of the invention.

The other four breast tumor tissue samples were isolated in the same way as above.

(2) Optimization of primary breast epithelial cell culture medium

Matrigel (registered trademark) (manufactured by BD Biosciences) was diluted at 1:100 in serum-free DMEM/F12 medium to prepare an extracellular matrix dilution, which was added to a 48-well culture plate with 200 μl/well to completely cover the bottom of the wells of the culture plate. The resultant was let stand for 1 hour in a 37°C incubator. After 1 hour, the extracellular matrix dilution was removed to provide a Matrigel-coated plate.

First, a basis medium was prepared. To a commercial DMEM/F-12 medium, GlutaMAX-I (manufactured by Thermo Fisher SCIENTIFIC) was added at the specified concentration (1:100 dilution), human insulin (manufactured by Sigma) was added at a final concentration of 10 μg/ml, ROCK kinase inhibitor Y27632 (manufactured by Sigma) was added at a final concentration of 10 μM, and penicillin-streptomycin (manufactured by Thermo Fisher SCIENTIFIC) was added at a 1:100 dilution ratio, which provides the basis medium.

Next, different types of additives (as shown in Table 1) were added to the basis medium to prepare breast epithelial cell culture mediums containing different supplementary components, and the mediums with different components were added at a volume of 500 μl/well to 48-well plates which were coated with extracellular matrix gel (Matrigel). Breast tumor cells (HMFL-XN1) isolated from breast cancer tissue in step (1) of this Example were inoculated in a Matrigel-coated 48-well culture plate at a cell density of $1 \times 10^4$ cells/well, and equal numbers of freshly isolated breast tumor cells (HMFL-XN1) were cultured under different medium formulations at 37°C, under 5% $CO_2$ concentration and 20% oxygen concentration. The medium was renewed every 4 days after the start of the culture. After 10 days of culture, cell counts were performed. As an experiment control, the basis medium without any additives was used. Breast tumor cells isolated from the remaining four breast cancer tissue samples were cultured and counted in the same manner as above. The experimental results are shown in Table 1.

[Table 1]

| Serial Number | Type of medium additives | Supplier | Concentration | Degree of cell proliferation |
|---|---|---|---|---|
| 1 | N2 | Gibco | 100X | + |
| 2 | Epidermal growth factor | R&D | 10ng/ml | + |
| 3 | Hepatocyte growth factor | Peprotech | 20ng/ml | + |
| 4 | Basic fibroblast growth factor | R&D | 20ng/ml | + |
| 5 | R-spondin1 | R&D | 250ng/ml | ∘ |
| 6 | R-spondin3 | R&D | 250ng/ml | ∘ |
| 7 | Prostaglandin E2 | Tocris | 1 μM | - |
| 8 | Insulin growth factor1 | Peprotech | 50ng/ml | + |
| 9 | Human leukemia inhibitory factor | Peprotech | 100ng/ml | - |
| 10 | Triiodothyronine | MCE | 5nM | ∘ |
| 11 | B27 | Gibco | 50X | + |
| 12 | A8301 | MCE | 500nM | + |
| 13 | SB202190 | MCE | 500nM | + |
| 14 | A8301+SB202190 | / | 500nM | + |
| 15 | Nicotinamide | Sigma | 10mM | - |
| 16 | N-Acetyl Cysteine | Sigma | 1.25mM | - |
| 17 | Nicotinamide + N-Acetyl Cysteine | / | 10mM+ 1.25mM | - |
| 18 | Blebbistatin | MCE | 10μM | - |
| 19 | Amphiregulin | R&D | 20ng/ml | + |

(continued)

| Serial Number | Type of medium additives | Supplier | Concentration | Degree of cell proliferation |
|---|---|---|---|---|
| 20 | Cholera toxin | Sigma | 0.1nM | ∘ |
| 21 | Fibroblast growth factor 7 | R&D | 10ng/ml | + |
| 22 | Fibroblast growth factor 10 | Peprotech | 10ng/ml | + |
| 23 | Neuregulin 1 | Peprotech | 10nM | + |
| 24 | Noggin | R&D | 100ng/ml | ∘ |
| 25 | Interleukin 6 | R&D | 5ng/ml | - |
| 26 | Interleukin 10 | R&D | 50ng/ml | ∘ |

Wherein, "+" indicates that compared with the basis medium, the medium added with the additive(s) can promote the proliferation of primary breast tumor epithelial cells isolated from at least three cases of breast cancer tissues; "-" indicates that the medium added with the additive(s) has the effect of inhibiting proliferation of primary breast tumor epithelial cells isolated from at least two cases of breast cancer tissues; "o" indicates that the medium added with this additive(s) has no significant effect on the proliferation of primary breast tumor epithelial cells isolated from at least three cases of breast cancer tissues.

[Example 2]

Culture of human primary breast epithelial cells

(1) Preparation of primary breast epithelial cell culture medium

First, a basis medium was prepared in the same manner as in step (2) of Example 1. To the basis medium, human amphiregulin (manufactured by R&D Systems) was added at a final concentration of 20 ng/ml, EGF (manufactured by Peprotech) was added at a final concentration of 10 ng/ml, B27 (manufactured by Thermo Fisher SCIENTIFIC) was added at a dilution ratio of 1:50, human Neuregulin 1 (manufactured by Peprotech) was added at a final concentration of 10 nM, FGF7 (manufactured by R&D Systems) was added at a final concentration of 10 ng/ml, the TGFβ1 inhibitor A8301 (manufactured by MCE) was added at a final concentration of 500 nM, and the P38/MAPK inhibitor SB202190 (manufactured by MCE) was added at a final concentration of 500 nM, to prepare a primary breast epithelial cell culture medium.

(2) Culture of primary breast tumor cells derived from breast cancer tissue and normal breast epithelial cells derived from paracancerous tissue

Breast epithelial cells derived from cancer tissue and breast epithelial cells derived from paracancerous tissue were isolated respectively from the cancer tissue and the paracancerous breast tissue of the same breast cancer patient, using the same method as in step (1) of Example 1. The paracancerous breast tissue samples were collected from the breast tissue at least 5 cm away from the breast cancer tissue. Next, the breast tumor cells derived from cancer tissue were counted with a hemocytometer, and then inoculated at a density of $4 \times 10^4$ cells/well into a 12-well plate coated with Matrigel (registered trademark) (manufactured by BD Biosciences). 2 mL of the prepared primary breast epithelial cell culture medium was added to the 12-well plate, which was cultured at 37°C under the conditions of 5% $CO_2$ concentration and 20% oxygen concentration.

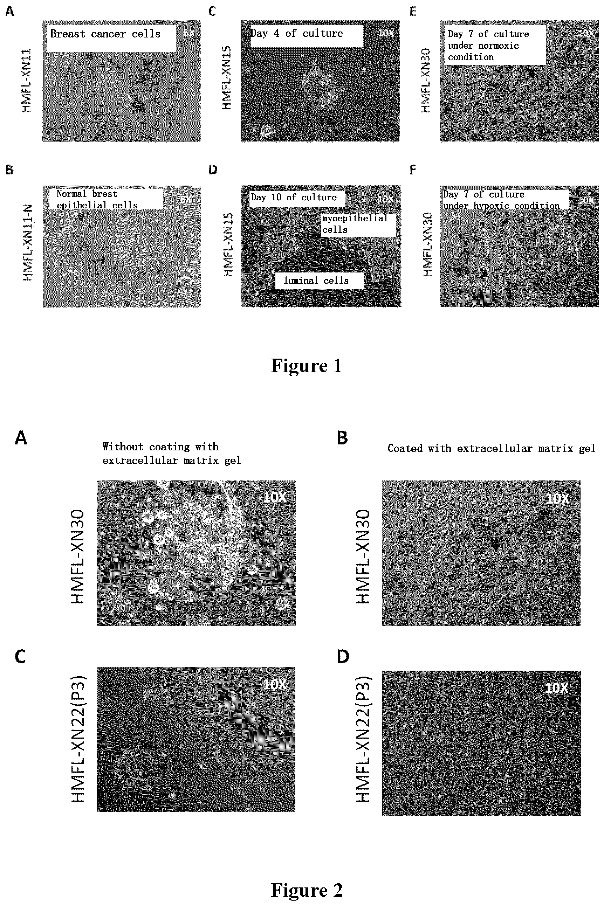

Figure 1(A) shows a microscopy image (under a 50x inverted phase contrast microscope) of breast tumor cells (HMFL-XN11) which were isolated from a surgically resected breast tumor sample and cultured to day 7 after inoculation. Microscopic observation shows that the cultured primary breast tumor cells derived from the cancer tissue were of high purity and did not contain interstitial cells such as fibroblasts and the like. Figure 1(B) shows a microscopy image (under a 50x inverted phase contrast microscope) of primary breast normal epithelial cells (HMFL-XN11-N) which were isolated from the paracancerous tissue samples of the same patient as HMFL-XN11, inoculated into a Matrigel-coated 12-well plate at a density of $4 \times 10^4$ cells/well and cultured to day 7 after inoculation. Figures 1(A) and 1(B) indicate that the use of the primary breast epithelial cell culture medium and culture method of the invention can achieve efficient culture *in vitro* for both breast tumor cells derived from breast cancer tissue and normal breast epithelial cells derived from paracancerous tissue. The cultured primary cells do not contain interstitial cells such as fibroblasts and the like.

Figures 1(C) and 1(D) are comparison of microscopy images (under a 100x inverted phase contrast microscope) of primary breast tumor cells (HMFL-XN15) cultured to day 4 after inoculation and cultured to day 10 after inoculation, respectively, wherein the primary breast tumor cells (HMFL-XN15) were isolated from another surgically resected breast cancer tissue samples and inoculated into a Matrigel-coated 12-well plate at a density of $4 \times 10^4$ cells/well. Figure 1(D) shows that two different cell subpopulations which constitute the breast gland, i.e., luminal cells and myoepithelial cells, can be cultured by using the primary cell culture medium and the culture method of the invention, and thus, achieves an *in vitro* culture that can preserve the heterogeneity of tumor sample. Comparing Figure 1(C) and 1(D), it can be confirmed that the breast cancer tissue samples are isolated, inoculated, and cultured using the medium and the culture method of the invention, which results in the formation of obvious breast epithelial cell clones on day 4 after inoculation and culture, and the exponential expansion of cell number on day 10 of culture, suggesting that the technology of the invention is an efficient technology for expanding breast epithelial cells *in vitro*.

(3) Culture of primary breast epithelial cells under normoxic and hypoxic conditions

The primary breast tumor cells (HMFL-XN30) derived from breast cancer tissue were isolated from the cancer tissue of a breast cancer patient, using the same method as step (1) of Example 1. Next, the primary breast tumor cells HMFL-XN30 with equal cell numbers ($4 \times 10^4$ cells/well) were inoculated into a Matrigel (registered trademark) (manufactured by BD Biosciences)-coated 12-well plate. 2 mL of the prepared primary breast epithelial cell culture medium was added to the 12-well plate, and equal numbers of breast tumor cells were cultured under the conditions of 20% oxygen concentration (normoxic condition) and 2% oxygen concentration (hypoxic condition), respectively. The micrograph photos (under a 100x inverted phase contrast microscope) were taken after cultured to day 7. Figures 1(E) and 1(F) are the comparison of cells obtained by culturing the breast epithelial cells derived from cancer tissue under normoxic and hypoxic conditions, respectively. From Figures 1(E) and 1(F), it can be confirmed that high-efficiency culture of the primary breast epithelial cells derived from cancer tissue can be achieved under both normoxic (20% oxygen concentration) and hypoxic conditions (2% oxygen concentration) when using the culture medium and the culture method of the invention.

(4) Comparison of the culture results of primary breast tumor cells derived from breast cancer tissue under the conditions of with and without coating of extracellular matrix gel

The primary breast tumor cells (HMFL-XN30, HMFL-XN22) derived from cancer tissues were isolated from the cancer tissues of two breast cancer patients, using the same method as step (1) of Example 1. Next, equal numbers ($4 \times 10^4$ cells/well) of the primary breast tumor cells HMFL-XN30 were inoculated into a Matrigel-coated (registered trademark) (manufactured by BD Biosciences) 12-well plate and a 12-well plate without any treatment, respectively. 2 mL of the prepared primary breast epithelial cell culture mediums were added to the 12-well plates, and equal numbers of primary breast tumor cells were cultured under the condition of 20% oxygen concentration. After cultured to day 10, photographs were taken. HMFL-XN22 (P3) were breast tumor cells HMFL-XN22 that were continuously cultured to the third passage under the conditions of Matrigel-coated and without Matrigel-coated, respectively, after isolation. The remaining steps are the same as those of HMFL-XN30.

Figure 2 are microscopy images of the breast cancer tissue-derived primary breast tumor cells HMFL-XN30 and HMFL-XN22 (P3) where were cultured to day 10 under the conditions of Matrigel-coated and without Matrigel-coated, respectively. According to Figure 2, it can be confirmed that the culture plate coated with Matrigel is more favorable for the proliferation of breast tumor cells than the culture plate without any treatment.

[Example 3]

Proliferation effect of amphiregulin on primary breast tumor cells derived from breast cancer tissue

(1) A primary breast epithelial cell culture medium was prepared in the same manner as in step (1) of Example 2. In addition, another primary medium without amphiregulin was prepared by removing amphiregulin from the formulation of the primary breast epithelial cell culture medium.
(2) By using the same method as step (1) of Example 1, primary breast tumor cells (HMFL-XN2, HMFL-XN12) derived from surgically resected cancer tissue samples of two different breast cancer patients and primary breast tumor cells (HMFL-XN13) derived from a puncture sample of a breast cancer patient were obtained.

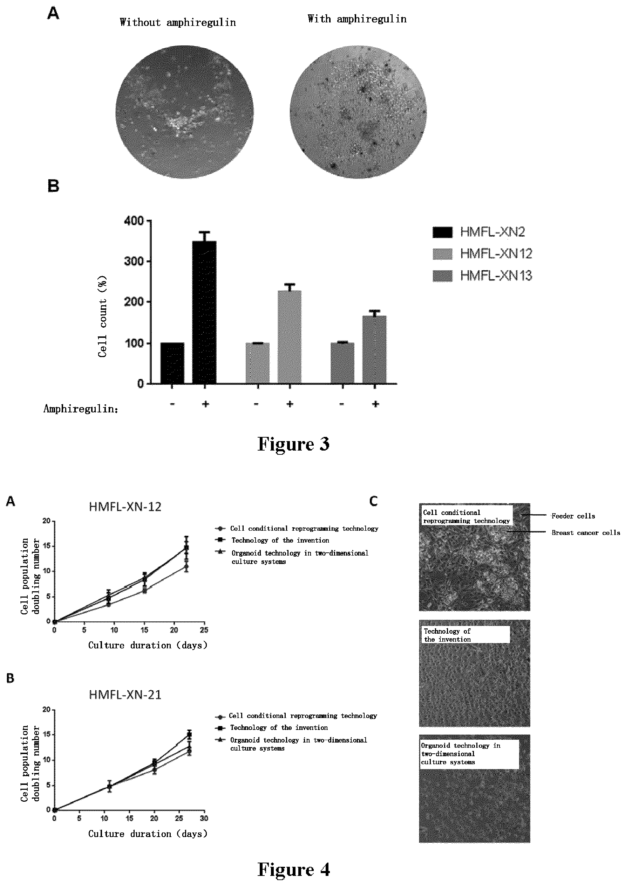

The primary breast tumor cells (HMFL-XN2) derived from surgically resected cancer tissue were inoculated at the same density ($5 \times 10^4$ cells/well) into a Matrigel (registered trademark) (manufactured by BD Biosciences)-coated 12-well plate. Cells were cultured at 37°C and 20% oxygen concentration in the primary breast epithelial cell medium of the invention containing amphiregulin and the primary medium without amphiregulin, with two replicate wells per group. Figure 3(A) shows the microscopy images (under a 100x inverted phase contrast microscope) of the tumor cell clones formed after culturing to day 6 in the primary culture medium without and with amphiregulin, wherein, the cultured cells (HMFL-XN2) were isolated from the same breast cancer patient and inoculated with the same cell numbers ($5 \times 10^4$ cells/well).

Figure 3(B) shows the results of cell count for three cases of breast cancer tissue-derived breast tumor cells (HMFL-XN2, HMFL-XN-12 and HMFL-XN13), after culturing to day 10 in the primary culture medium without amphiregulin and with amphiregulin, respectively, with two replicate wells in each group. It can be seen from Figure 4 that taking the group of the cells cultured in the culture medium without amphiregulin as a control, the addition of amphiregulin can promote the proliferation of the primary breast epithelial cells, which may promote the proliferation of the primary breast epithelial cells by at least 50% and up to 250%.

[Example 4]

Continuous culture of primary breast tumor cells derived from breast cancer tissue and drawing of the growth curve for the same

(1) The primary breast epithelial cell culture medium was prepared in the same manner as in step (1) of Example 2. As a control, another culture medium for cell conditional reprogramming technology (hereinafter also referred to as "cell conditional reprogramming medium") was prepared. For the preparation steps, please refer to Liu et al., Nat Protoc., 12(2): 439-451, 2017. The formulation of the culture medium is shown in Table 2. In addition, as another control, a culture medium for breast gland organoids (hereinafter also referred to as "organoid medium") was prepared. For the preparation steps, see Hans Clevers et al., Cell, 11; 172(1-2): 373-386, 2018, and the formulation of the culture medium is shown in Table 3.

[Table 2] Composition of cell conditional reprogramming medium

| Medium composition | Supplier | Article number | Final concentration |
|---|---|---|---|
| DMEM medium | Corning | 10-013-CVR | 65% |
| Fetal bovine serum | Gibco | 16140-071 | 10% |
| Ham's F12 Nutrient Mixture | Gibco | 11765-054 | 25% |
| Hydrocortisone | Sigma-Aldrich | H-0888 | 25 ng/ml |
| Epidermal growth factor | R&D | 236-EG | 0.125 ng/ml |
| Insulin | Sigma-Aldrich | I-5500 | 5 $\mu$g/ml |
| Amphotericin B | Sigma-Aldrich | V900919 | 250 ng/ml |
| Gentamicin | Gibco | 15710-064 | 10 $\mu$g/ml |
| Cholera Toxin | Sigma-Aldrich | C8052 | 0.1 nM |
| Y27632 | Enzo | 270-333M025 | 10 $\mu$M |

[Table 3] Composition of organoid medium

| Medium composition | Supplier | Article number | Final concentration |
|---|---|---|---|
| R-Spondin 1 conditioned medium | prepared | / | 10% |
| R-Spondin 3 recombinant protein | R&D | 3500-RS/CF | 250 ng/ml |
| Neuregulin 1 | Peprotech | 100-03 | 5 nM |
| Fibroblast growth factor 7 | Peprotech | 100-19 | 5 ng/ml |
| Fibroblast growth factor 10 | Peprotech | 100-26 | 20 ng/ml |
| Epidermal growth factor | Peprotech | AF-100-15 | 5 ng/ml |
| Noggin | Peprotech | 120-10C | 100 ng/ml |
| A83-01 | Tocris | 2939 | 500 nM |

(continued)

| Medium composition | Supplier | Article number | Final concentration |
|---|---|---|---|
| Y-27632 | Abmole | Y-27632 | 5 mM |
| SB202190 | Sigma | S7067 | 500 nM |
| B27 | Gibco | 17504-44 | 1x |
| N-Acetyl Cysteine | Sigma | A9165-5g | 1.25 mM |
| Nicotinamide | Sigma | N0636 | 5 mM |
| Glutamine additive 100x | Invitrogen | 12634-034 | 1x |
| Hepes buffer | Invitrogen | 15630-056 | 10 mM |
| Penicillin/Streptomycin | Invitrogen | 15140-122 | 100 U/ml |
| Primocin | Invitrogen | Ant-pm-1 | 50 mg/ml |
| Advanced DMEM/F12 Medium | Invitrogen | 12634-034 | 1x |

(2) By using the same method as step (1) of Example 1, the primary breast tumor cells (HMFL-XN12 and HMFL-XN21) derived from two cases of breast cancer tissues were obtained. Next, HMFL-XN12 and HMFL-XN21 were cultured at the same density ($4 \times 10^4$ cells/well) under the following three culture conditions:

A. The technology of the invention: the primary breast tumor cells were inoculated into a 12-well plate coated with Matrigel (registered trademark) (manufactured by BD Biosciences) at a density of $4 \times 10^4$ cells/well, cultured using 2 mL of the primary breast epithelial cell culture medium of the invention;

B. Cell conditional reprogramming technology: the primary breast tumor cells were inoculated onto a $\gamma$-ray irradiated mouse fibroblast cell line J2 cells (purchased from Kerafast) at a density of $4 \times 10^4$ cells/well, cultured in a 12-well plate using the cell conditional reprogramming medium (the detailed steps, see Liu et al., Am J Pathol, 183(6): 1862-1870, 2013);

C. The primary breast tumor cells were inoculated into a Matrigel (registered trademark) (manufactured by BD Biosciences)-coated 12-well plate at a density of $4 \times 10^4$ cells/well, and cultured in the 12-well plate using 2 mL of organoid medium.

In the above three cultures, the cells cultured under the three culture conditions wherein the mediums were renewed every 4 days.

(3) For the primary breast tumor cells (HMFL-XN12 and HMFL-XN21) cultured by the technology of the invention, when the growth of the cells in the culture plate covers about 80% of the bottom area of the plate, the supernatant of the culture medium in the 12-well plate was discarded, and 1 mL of 0.05% trypsin (Thermo Fisher: 25300062) was added to digest the cells; after incubating at 37°C for 15 minutes, the digested cells were resuspended in 5 mL of DMEM/F12 medium containing 10% (v/v) fetal bovine serum, 100 U/mL penicillin and 100 U/mL streptomycin, and the resultant was collected into a centrifuge tube for centrifuging at 350 g/min for 5 minutes; the centrifuged cell precipitations were resuspended in the culture medium of the invention, and the cells were count with a hemocytometer; the cells were inoculated at a density of $4 \times 10^4$ cells/well into another 12-well culture plate coated with extracellular matrix gel for further culture.

The cells cultured under the other two culture conditions were digested, passaged and counted in the same manner as above, and the cells were inoculated respectively according to step B or step C described in step (2) of Example 4.

When the passaged cells grew in the culture plate to cover about 80% of the bottom area of the plate again, the cultured cells were digested, collected and counted according to the above operating method. The cells were inoculated at a density of $4 \times 10^4$ cells/well again and cultured continuously.

The following is the formula for calculating the cell population doubling number of primary breast epithelial cells under different culture conditions:

$$\text{Cell population doubling number} = [\log(N/X_0)]/\log 2$$

where N is the number of cells for the passage and $X_0$ is the number of cells at initial inoculation (see Greenwood et al.,

Environ Mol Mutagen 2004, 43(1): 36-44).

Figures 4(A) and 4(B) are the growth curves of two cases of cells under three different culture conditions drawn by Graphpad Prism7.0 software, which take the culture days as the abscissa and the cell population doubling number as the ordinate. From Figures 4(A) and 4(B), it can be confirmed that the proliferation rate of breast epithelial cells cultured by the technology of the invention is higher than that of the cell conditional reprogramming technology, and is comparative to that of the organoid culture technology.

The photos of the cells in Figure 4(C) are a microscopy images (under a 50x inverted phase contrast microscope) of HMFL-XN21 cultured to the third passage (day 27) under three different culture conditions.

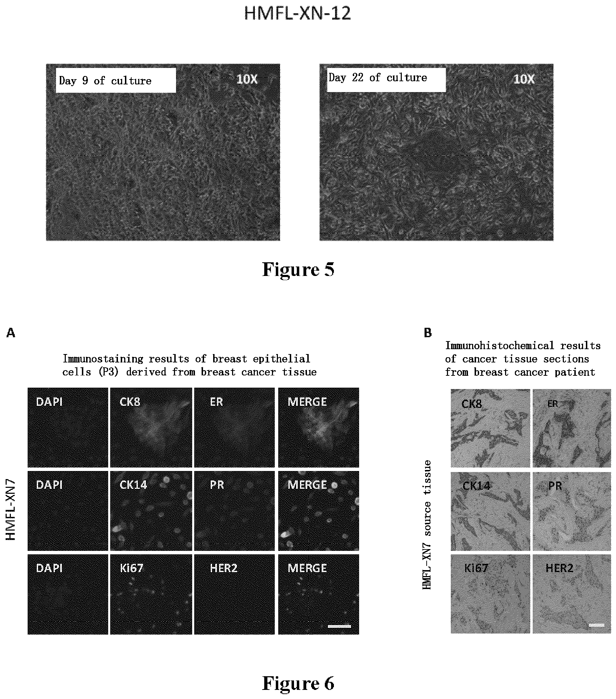

Figure 5 is a comparison of the microscopy images (under a 100x inverted phase contrast microscope) of HMFL-XN12 cultured to day 9 and day 22 with the culture medium of the invention. It can be confirmed from Figure 5 that the technology of the invention can continuously culture the primary breast epithelial cells, and the morphology of the cells after multiple passages and continuous culture does not change significantly compared with that before passage.

[Example 5]

Identification of immune markers of the tumor tissue-derived primary breast tumor cell

(1) A primary breast tumor cell culture medium was prepared in the same manner as in step (1) of Example 2.

(2) Cancer tissue about the size of a soybean was taken from a surgical resection sample of a breast cancer patient, and immersed in 10 mL of 4% paraformaldehyde. Breast epithelial cells (HMFL-XN7) were obtained from the remaining cancer tissue in the same manner as in step (1) of Example 1. HMFL-XN7 was continuously cultured to the third passage using the same method as described in step (3) of Example 4.

(3) Immunofluorescence assay was used to detect the expression of important cancer-related biomarkers on human breast tumor cells. The primary antibodies used were CK8 (manufactured by Abcam), CK14 (manufactured by Abcam), ER (manufactured by Cell Signaling Technology), PR (manufactured by Cell Signaling Technology), HER2 (manufactured by Cell Signaling Technology), and Ki67 (manufactured by Cell Signaling Technology). The secondary antibodies used were Anti-Rabbit IgG (H+L), F(ab')2 Fragment (Alexa Fluor® 488 Conjugate) (manufactured by Cell Signaling Technology), Anti-Mouse IgG (H+L), F(ab')2 Fragment (Alexa Fluor® 594 Conjugate) (manufactured by Cell Signaling Technology). ER and PR are important indicators to predict whether the patients can accept the endocrine therapy; HER2 is an important indicator to predict whether the patients can accept anti-HER2 targeted therapy; Ki67 is an indicator to judge the malignancy and prognosis of breast cancer; CK8 and CK14 are biomarkers for epithelial cells, which are also used in the identification and diagnosis of breast cancer.

The breast tumor cells (HMFL-XN7) cultured to the third passage according to the technology of the invention were inoculated on a glass slide coated with Matrigel, and the coating method was the same as the step (2) of Example 1. The cells were cultured by using the primary breast epithelial cell culture medium of the invention, and the cells were grown on the glass slide. After being rinsed twice with PBS buffer, the cells were fixed with 4% paraformaldehyde for 15 minutes, and then incubated with 1% BSA, 1% Triton X-100 in TBST (TBS + 0.1% Tween 20) at room temperature for one hour. The cells were rinsed with PBS buffer 3 times for 3 minutes each time. PBS solution was removed; 50 $\mu$L of the primary antibody diluent (prepared according to the antibody instructions) was dropped on the slide; the cells were incubated at room temperature for 60 minutes, and then rinsed 3 times with PBS for 3 minutes each time; the secondary antibody diluent (prepared according to the antibody instructions) was added dropwise; the cells were incubated at room temperature for 60 minutes, and then rinsed 3 times with PBS for 3 minutes each time. The cells were incubated with 1 $\mu$g/mL of DAPI dye (manufactured by Sigma) for 10 minutes, and then rinsed once with PBS. After sealing the coverslip with a drop of mounting medium (manufactured by Thermo Fisher Scientific), the expression of breast cancer-related biomarkers ER, PR, HER2, CK8, CK14 and Ki67 on the cells was photographed (under a 400x fluorescence microscope). The results were shown in Figure 6(A). The results of immunofluorescence staining show that the number of ER(+), PR(+), HER2(-), and Ki67 positive cells in the breast epithelial cells cultured to the third passage by the technology of the invention was about 20%.

(4) The expression of important biomarkers related to the breast cancer in the original tissue, from which HMFL-XN7 cells were derived, was detected by immunohistochemistry. The tissue was fixed with 4% paraformaldehyde, embedded in paraffin, and cut into tissue sections of 4 $\mu$m thickness with a microtome. Routine immunohistochemical detection was then performed (for detailed steps, see Yu et al., Science, 345(6193): 216-220, 2014). The primary antibodies used were the same as those used in immunofluorescence. The expression of cancer-related biomarkers ER, PR, HER2, CK8, CK14 and Ki67 on the cells was photographed (under a 200x biological microscope), and the results are shown in Figure 6(B). The immunohistochemistry results for the patient show that ER(+), PR(+), HER2(-), and Ki67 positive cell number was about 20%.

Figures 6(A) and 6(B) confirm that the expression of breast cancer-related biomarkers on the cells that were cultured from the breast tumor cells (HMFL-XN7) by the technology of the invention to the third passage, was consistent with the expression of markers on the original tissue section from which the cells were derived. This suggests that the cells cultured by the technology of the invention maintain the original pathological characteristics of the cancer tissues of the breast cancer patients.

[Example 6]

Gene mutation and karyotype analysis of primary breast tumor cells derived from cancer tissue

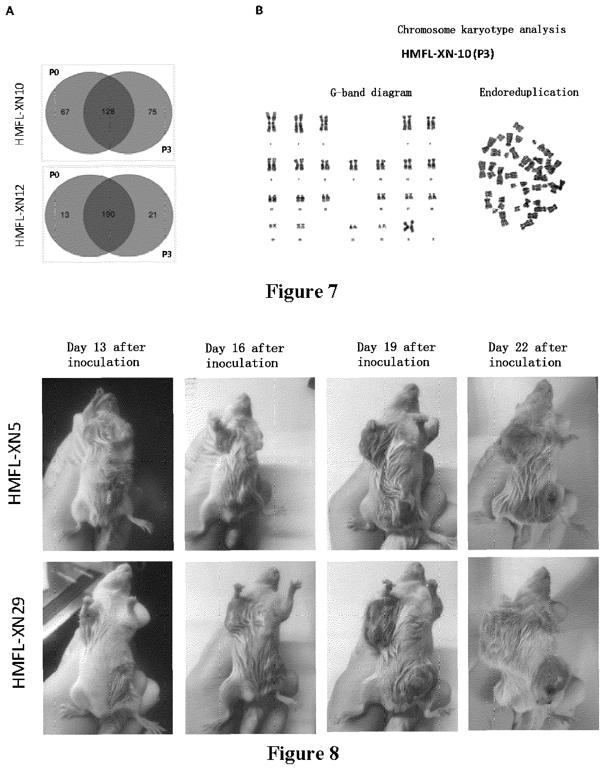

(1) Gene mutation analysis: the breast tumor cells (HMFL-XN10, HMFL-XN12) were continuously cultured by the method of the invention in accordance with step (3) of Example 4; the breast tumor cells (P3) cultured *in vitro* for the third passage and the non-passaged cancer tumor cells (P0) directly derived from the breast cancer patient were collected by centrifugation, and the genomic DNA of the cells was extracted using DNeasy blood & tissue kit (manufactured by QIAGEN). 2 mL of peripheral blood was collected from the patient who donates the cells, and the genomic DNA was extracted by the same method as a background control. Subsequently, whole-exome sequencing was performed on the genomic DNA of the cells and the blood samples (for detailed procedures, see Hans Clevers et al., Cell, 11; 172(1-2): 373-386, 2018), and the sequencing results were analyzed for tumor high-frequency mutated gene. The MuSiC software was used to analyze the high-frequency mutation of tumor. MuSiC took the gene mutation in the peripheral blood of the patient who donates the tumor sample as the background, performed statistical tests on each mutation type on the gene, and detected the gene with a significantly higher mutation rate than the background. The analysis results are shown in Figure 7(A). The Venn diagrams in Figure 7(A) show the alignments of the number of high-frequency mutation genes carried by breast tumor cells of different passages cultured by using the technology of the invention. HMFL-XN10 (P3) represents a high-frequency mutation analysis for the breast tumor cells from the breast cancer patient which have been cultured to the third passage by the culture method of the invention; HMFL-XN10 (P0) represents an analysis of initially isolated non-passaged breast tumor cells derived from the same breast cancer patient. The above analysis results were produced using the software available at https://bioinfogp.cnb.csic.es/tools/venny/index.html. The results of tumor high-frequency mutation analysis show that the high-frequency mutation genes of the two groups were substantially the same. HMFL-XN12 and HMFL-XN10 cells were operated in parallel using the same method. Figure 7(A) confirms that the cancer tissue-derived breast tumor cells cultured by the technology of the invention can maintain the original gene mutation characteristics of the cancer tissue of the patient.

(2) Chromosome karyotype analysis: the breast tumor cells (HMFL-XN10) were continuously cultured to the third passage (P3) using the same method of the invention according to step (3) of Example 4. One day before collection of the cells, 0.1 $\mu$g/mL of colchamine (manufactured by Gibco) was added to the culture medium; after 16 hours, the cells were digested, collected, and treated with 75 mM low-permeability KCI, and then were fixed with a 3:1 methanol:glacial acetic acid solution for 30 minutes. The fixed cells were evenly dropped on a glass slide and allowed to dry, and the cell nuclei were stained with 5 mg/ml DAPI (manufactured by Sigma) for 15 minutes; the cell metaphase were photographed by using a confocal laser microscope (purchased from Leica), and chromosomes were aligned in pairs.

Figure 7(B) shows that the chromosomal morphology and number of breast tumor cells cultured to the third passage by using the technology of the invention for HMFL-XN10 are normal, without the appearance of polyploidy or hypoploidy; however, the chromosomes in the nucleus were duplicated twice, and several chromosomes were close to each other, which was consistent with the karyotype of tumor cells, suggesting that the cells have abnormal chromosomes and have tumor cell characteristics. Figure 7(B) confirms that the cancer tissue-derived breast tumor cells cultured by the culture method of the invention can maintain the chromosome karyotype characteristics of the breast cancer patient.

[Example 7]

Xenograft tumorigenesis experiments of cancer tissue-derived primary breast tumor cells in mice

Breast tumor cells (HMFL-XN5, HMFL-XN29) were isolated and obtained from the cancer tissues of two cases of pathologically diagnosed triple-negative breast cancer patients by using the same method as step (1) in Example 1. HMFL-XN5 and HMFL-XN29 were cultured respectively according to the method of step (2) of Example 2, and when the number of breast tumor cells reached $1 \times 10^7$, the breast tumor cells were digested and collected by using the same method of the invention as described in step (3) of Example 4. The breast tumor cell culture medium of the invention and Matrigel (registered trademark) (manufactured by BD Biosciences) were mixed at a ratio of 1:1, and 100 $\mu$L of the culture medium mixed with Matrigel was used to resuspend $5 \times 10^6$ breast tumor cells, and the resultant was injected into the breast fat pad and the axilla of the right forelimb of 6-week-old female highly immunodeficient mice (NCG) (purchased from Nanjing Model Animal Research Center), respectively. The volume and growth rate of tumors in mice generated from the breast tumor cells were observed and photographed every three days.

Figure 8 confirms that tumor formation can be observed in the two tumor cell inoculation sites of the mice on day 13 after tumor cell inoculation. From day 13 to day 22, the tumor proliferation in mice was obvious. This indicates that the cancer tissue-derived breast tumor cells cultured by the culture method of the invention have tumorigenicity in mice.

[Example 8]

Drug sensitivity functional test of cancer tissue-derived breast tumor cells

Taking a surgical resection sample from a breast cancer patient as an example, it is verified that the breast tumor cells cultured from the patient-derived breast tumor samples can be used to test the sensitivity of the tumor cells of the patient to different drugs.

1. Plating of primary breast tumor cells: the single cell suspension of breast tumor cells (HMFL-XN5, HMFL-XN7, HMFL-XN10, HMFL-XN12, and HMFL-XN29) obtained by the same method of the invention according to step (3) of Example 4, was inoculated at a density of 3000-5000 cells/well in a 384-well plate, and the cells were adhered overnight.
2. Drug gradient experiments:

(1) The drug storage plates were prepared by gradient dilution method: 10 $\mu$L of the drug stock solutions to be tested (the concentration of the drug stock solution was determined based on 2 times of the maximum blood concentration Cmax of the drug in the human body) were respectively taken, and added to 0.5 mL EP tubes containing 20 $\mu$L of DMSO; 10 $\mu$L of solutions from the above EP tubes were pipetted into second 0.5 mL EP tubes loaded with 20 $\mu$L of DMSO, that is, diluting the drugs in a ratio of 1:3. The above method was repeated for gradually dilution and 7 concentrations required for dosing were obtained. Different concentrations of drugs were added to the 384-well drug storage plates. Equal volume of DMSO was added to each well of the solvent control group as a control. In this example, the drugs to be tested were epirubicin (manufactured by MCE), lapatinib (manufactured by MCE), docetaxel (manufactured by MCE), and tamoxifen (manufactured by MCE).
(2) Using a high-throughput automated workstation (purchased from Perkin Elmer), different concentrations of drugs and solvent controls in the 384-well drug storage plates were added to 384-well cell culture plates plated with the breast tumor cells. The drug groups and the solvent control groups were each arranged with 3 duplicate wells. The volume of drugs added to each well was 100 nL.
(3) Test of cell viability: 72 hours after administration, Cell Titer-Glo assay kit (manufactured by Promega) was used to detect the chemiluminescence value of the cultured cells after drug administration. The magnitude of the chemiluminescence value reflects the cell viability and the effect of the drug on the cell viability. The prepared Cell Titer-Glo detection solution was added to each well, and a microplate reader was used to detect the chemiluminescence value after mixing.

Graphs were made by using Graphpad Prism 7.0 software and the half-inhibition rate $IC_{50}$ was calculated.

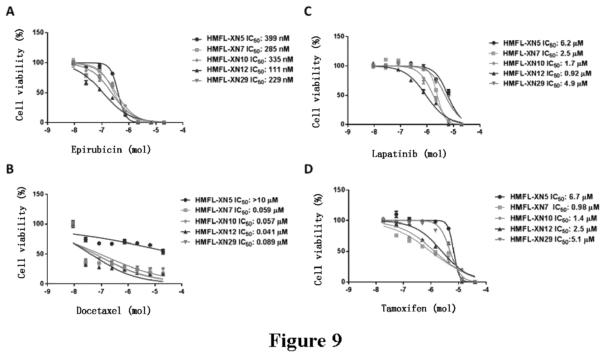

(4) The drug sensitivity testing results are shown in Figure 9.

Figures 9(A) to 9(D) respectively represent the sensitivity of the breast tumor cells cultured from surgically resected cancer tissue samples of five different breast cancer patients to two chemotherapeutic drugs epirubicin and docetaxel, to endocrine therapy drug tamoxifen, and to targeted drug lapatinib. The results show that the cells from the same patient have different sensitivities to different drugs, and the cells from different patients also have different sensitivities to the same drug.

Specifically, the breast tumor cells (HMFL-XN7) derived from a hormone receptor-positive, HER2 receptor-negative breast cancer patient were more sensitive to the endocrine therapy drug tamoxifen than other patients, with a half-inhibition rate of 0.98 $\mu$M; however, the sensitivity to the HER2-targeting drug lapatinib was lower, with a half-inhibition rate of 2.5 $\mu$M. The test results of the breast tumor cells (HMFL-XN12) derived from a HER2-positive breast cancer patient show that the cells were more sensitive to the anti-HER2 targeting drug lapatinib than other patients, with a half-inhibitory amount of 0.92 $\mu$M. In addition, the breast tumor cells (HMFL-XN29) derived from a triple-negative breast cancer patient were less sensitive to the endocrine therapy drug tamoxifen and the anti-HER2 targeting drug lapatinib than the breast tumor cells from other patients. On the other hand, the breast tumor cells (HMFL-XN5) from another triple-negative breast cancer patient were sensitive to none of the four testing drugs.

Figure 9 confirms that the sensitivity test results of the breast tumor cells, which were cultured by the invention from the cancer tissues of breast cancer patients, to chemotherapeutic drugs and targeted drugs, were consistent with the clinicopathological molecular typing of the patients, suggesting that the breast tumor cells cultured by the technology of the invention have potential uses in predicting the clinical efficacy of the breast cancer patients.

Although the invention has been described in details above with general description and specific embodiments, some modifications or improvements can be made on the basis of the invention, which would be obvious to those skilled in the art. Therefore, these modifications or improvements made without departing from the spirit of the invention should be within the protection scope of the invention.

Industrial applicability

The invention provides a culture medium and a culture method for culturing or expanding primary breast epithelial cells *in vitro.* The cell models obtained from the culture medium and the culture method of primary breast epithelial cells of the invention can be used to evaluate or screen the drugs for treating breast diseases.

## Claims

1.   A primary cell culture medium for culturing primary breast epithelial cells, **characterized in that**: the medium comprises amphiregulin.

2.   The primary cell culture medium of claim 1, **characterized in that**:
the content of the amphiregulin is 10 ng/ml to 100 ng/ml.

3.   The primary cell culture medium of claim 1, **characterized in that**:
the medium further comprises one or more or all of epidermal growth factor, insulin, B27, ROCK kinase inhibitor Y27632, Neuregulin 1, fibroblast growth factor 7, TGFβ type I receptor inhibitor A8301, and P38/MAPK inhibitor SB202190.

4.   The primary cell culture medium of claim 3, **characterized in that**:

the content of epidermal growth factor is 2.5 ng/ml or more, preferably 2.5 ng/ml to 20 ng/ml;
the content of insulin is 1 μg/ml to 10 μg/ml;
B27 is diluted with a final concentration of 1:25 to 1:100;
the content of Y27632 is 5 μM to 15 μM;
the content of Neuregulin 1 is 5 nM to 20 nM;
the content of fibroblast growth factor 7 is 2.5 ng/ml to 20 ng/ml;
the content of A8301 is 100 nM to 500 nM;
the content of SB202190 is 100 nM to 500 nM.

5.   The primary cell culture medium of claim 1, **characterized in that**:
the medium does not comprise serum, bovine pituitary extract, Wnt agonists, R-spondin family proteins, BMP inhibitors, fibroblast growth factor 10, nicotinamide and N-acetylcysteine.

6.   The primary cell culture medium of any one of claims 1 to 5, **characterized in that**:
the primary breast epithelial cells are breast tumor cells, normal breast epithelial cells, or breast epithelial stem cells.

7.   A culture method for primary breast epithelial cell, **characterized in that**, comprising the following steps:

(1) preparing the primary cell culture medium according to any one of claims 1 to 6;
(2) coating a culture vessel with extracellular matrix gel diluent;
(3) inoculating primary breast epithelial cells in the coated culture vessel, culturing the cells under normoxic condition or hypoxic condition by using the primary cell culture medium, and digesting the cells for passaging when the primary breast epithelial cells grow to a cell density that accounts for 80% to 90% of the bottom area of the culture vessel.

8.   The culture method of claim 7, **characterized in that**:

the extracellular matrix gel is of low growth factor type;

the extracellular matrix gel is diluted with serum-free medium, and the dilution ratio of the extracellular matrix gel is 1:50 to 1:400;

the coating step involves adding the diluted extracellular matrix gel into the culture vessel to cover the bottom of the culture vessel completely, and standing for 30 minutes or more.

9. A method for evaluating the efficacy of the drug for treating breast diseases, **characterized in that**, comprising the following steps:

(1) culturing breast epithelial cells by using the culturing method according to claim 7 or 8;

(2) selecting the drugs for testing;

(3) based on the maximum plasma concentration of the drug Cmax as a reference, taking 2-5 times of Cmax as the initial concentration, and diluting the drug into different drug concentration gradients;

(4) digesting the breast epithelial cells cultured in step (1) into a single cell suspension, diluting the single cell suspension with the primary cell culture medium according to any one of claims 1 to 6 containing extracellular matrix gel, adding the diluted cell suspension to the multi-well plate at the density of 1000-10000 cells per well, and adhering overnight;

(5) adding the drug with gradient dilutions to the adherent cells obtained in step (4);

(6) detecting the cell viability.

10. The method of claim 9, **characterized in that**:

in the cell viability detection, a cell viability detection reagent is added to each well; and after uniformly shaking, the chemiluminescence intensity of each well is measured with a fluorescence microplate reader; a drug dose-effect curve is plotted based on the measured values; and the inhibitory intensity of each drug on the proliferation of cells is calculated.

Figure 1

Figure 2

**Figure 3**

**Figure 4**

HMFL-XN-12

**Figure 5**

A

Immunostaining results of breast epithelial
cells (P3) derived from breast cancer tissue

B

Immunohistochemical results
of cancer tissue sections
from breast cancer patient

**Figure 6**

A

B

Chromosome karyotype analysis

**HMFL-XN-10(P3)**

G-band diagram

Endoreduplication

## Figure 7

|  | Day 13 after inoculation | Day 16 after inoculation | Day 19 after inoculation | Day 22 after inoculation |
| --- | --- | --- | --- | --- |

## Figure 8

Figure 9

<table>
<tr><td colspan="2" align="center">**INTERNATIONAL SEARCH REPORT**</td><td>International application No.<br><br>**PCT/CN2019/119116**</td></tr>
</table>

**A.    CLASSIFICATION OF SUBJECT MATTER**

   C12N 5/095(2010.01)i;  C12N 5/00(2006.01)i;  C12N 5/071(2010.01)i

According to International Patent Classification (IPC) or to both national classification and IPC

**B.    FIELDS SEARCHED**

Minimum documentation searched (classification system followed by classification symbols)

   C12N

Documentation searched other than minimum documentation to the extent that such documents are included in the fields searched

Electronic data base consulted during the international search (name of data base and, where practicable, search terms used)

   DWPI, SIPOABS, CNABS, USTXT, JPTXT, EPTXT, WOTXT, CNTXT, cnki, pubmed, isi web of science: 乳腺, 上皮细胞, 表皮生长因子, 胰岛素, 神经调节素1, 双向调节素, 双条蛋白, 成纤维细胞生长因子7, 培养基, 牛垂体, 烟酰胺, N-乙酰半胱氨酸, 细胞外基质, 胶, Amphiregulin, NRG1, a8301, medium, breast, Mammary, AREG, epithelial cells, SB202190, egf, insulin, NRG-1, fgf7, bovine pituitary, Y27632, B27, bovine pituitary, Nicotinamide, N-acetylcysteine, extracellular matrix, ecm, gel

**C.    DOCUMENTS CONSIDERED TO BE RELEVANT**

| Category* | Citation of document, with indication, where appropriate, of the relevant passages | Relevant to claim No. |
|---|---|---|
| X | WO 2016083612 A1 (KONINKLIJKE NEDERLANDSE AKADEMIE VAN WETENSCHAPPEN) 02 June 2016 (2016-06-02)<br>   see claims 1-30, description page 34 last paragraph - page 36 paragraph 1, page 36 line 30- page 37, page 38 lines 22-28, page 47 lines 15-18, page 53 lines 24-26, page 56 line 10- page 57 lines 1-2, line 2 | 1-4, 6-10 |
| X | WO 2016083613 A2 (KONINKLIJKE NEDERLANDSE AKADEMIE VAN WETENSCHAPPEN) 02 June 2016 (2016-06-02)<br>   see claims 1-16, 23-30, description page 53 lines 8-12, page 68 paragraph 2, page 73 lines 29-31, pages 85-86, page 87 paragraph 3 | 1-4, 6-10 |
| X | WO 2016180421 A1 (UNIV COPENHAGEN) 17 November 2016 (2016-11-17)<br>   see claims 1-47 | 1-4, 6-10 |
| A | US 5830995 A (BRISTOL-MYERS SQUIBB COMPANY) 03 November 1998 (1998-11-03)<br>   see entire document | 1-10 |

☑ Further documents are listed in the continuation of Box C.      ☑ See patent family annex.

| | |
|---|---|
| *    Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |
| Date of the actual completion of the international search<br><br>**28 July 2020** | Date of mailing of the international search report<br><br>**17 August 2020** |
| Name and mailing address of the ISA/CN<br><br>**China National Intellectual Property Administration (ISA/CN)**<br>**No. 6, Xitucheng Road, Jimenqiao Haidian District, Beijing 100088**<br>**China** | Authorized officer |
| Facsimile No. **(86-10)62019451** | Telephone No. |

Form PCT/ISA/210 (second sheet) (January 2015)

International application No.

**PCT/CN2019/119116**

**INTERNATIONAL SEARCH REPORT**

## C. DOCUMENTS CONSIDERED TO BE RELEVANT

| Category* | Citation of document, with indication, where appropriate, of the relevant passages | Relevant to claim No. |
|---|---|---|
| X | Chandrani Mukhopadhyay et al. "Distinct Effects of EGFR Ligands on Human Mammary Epithelial Cell Differentiation" *PLoS One,* Vol. 8, No. 10, 04 October 2013 (2013-10-04), ISSN: 1932-6203, e75907, see page 2 left-hand column last paragraph | 1-4, 6 |

Form PCT/ISA/210 (second sheet) (January 2015)

**INTERNATIONAL SEARCH REPORT**
Information on patent family members

International application No.

**PCT/CN2019/119116**

| Patent document cited in search report | | | Publication date (day/month/year) | Patent family member(s) | | | Publication date (day/month/year) |
|---|---|---|---|---|---|---|---|
| WO | 2016083612 | A1 | 02 June 2016 | AU | 2015352350 | A1 | 15 June 2017 |
| | | | | GB | 201421094 | D0 | 14 January 2015 |
| | | | | CA | 2968751 | A1 | 02 June 2016 |
| | | | | US | 2017342385 | A1 | 30 November 2017 |
| | | | | EP | 3224351 | A1 | 04 October 2017 |
| | | | | JP | 2017536827 | A | 14 December 2017 |
| WO | 2016083613 | A2 | 02 June 2016 | AU | 2015352351 | A1 | 15 June 2017 |
| | | | | GB | 201507834 | D0 | 17 June 2015 |
| | | | | US | 2017275592 | A1 | 28 September 2017 |
| | | | | GB | 2532814 | A | 01 June 2016 |
| | | | | WO | 2016083613 | A3 | 18 August 2016 |
| | | | | JP | 2018503360 | A | 08 February 2018 |
| | | | | CA | 2968736 | A1 | 02 June 2016 |
| | | | | EP | 3224346 | A2 | 04 October 2017 |
| | | | | GB | 201421092 | D0 | 14 January 2015 |
| WO | 2016180421 | A1 | 17 November 2016 | EP | 3294874 | A1 | 21 March 2018 |
| | | | | US | 2018135010 | A1 | 17 May 2018 |
| | | | | CA | 2985873 | A1 | 17 November 2016 |
| US | 5830995 | A | 03 November 1998 | AU | 2876989 | A | 24 August 1989 |
| | | | | JP | H02104596 | A | 17 April 1990 |
| | | | | NO | 177788 | C | 22 November 1995 |
| | | | | DK | 29989 | A | 26 July 1989 |
| | | | | IT | 8919186 | D0 | 25 January 1989 |
| | | | | HU | T52545 | A | 28 July 1990 |
| | | | | NO | 177788 | B | 14 August 1995 |
| | | | | SE | 8900247 | L | 26 July 1989 |
| | | | | GB | 2214185 | B | 17 June 1992 |
| | | | | NZ | 227729 | A | 25 February 1992 |
| | | | | CN | 1038126 | A | 20 December 1989 |
| | | | | AT | 399720 | B | 25 July 1995 |
| | | | | GB | 2214185 | A | 31 August 1989 |
| | | | | IT | 1229504 | B | 03 September 1991 |
| | | | | DK | 29989 | D0 | 24 January 1989 |
| | | | | FR | 2628109 | A1 | 08 September 1989 |
| | | | | NO | 890299 | L | 26 July 1989 |
| | | | | SE | 504554 | C2 | 03 March 1997 |
| | | | | IE | 61146 | B1 | 05 October 1994 |
| | | | | FI | 890312 | A0 | 20 January 1989 |
| | | | | FI | 890312 | A | 26 July 1989 |
| | | | | AU | 623109 | B2 | 07 May 1992 |
| | | | | NO | 890299 | A | 26 July 1989 |
| | | | | IE | 890207 | L | 25 July 1989 |
| | | | | NL | 8900178 | A | 16 August 1989 |
| | | | | AT | A14789 | A | 15 November 1994 |
| | | | | DE | 3902157 | A1 | 27 July 1989 |
| | | | | IL | 89033 | A | 30 May 1994 |
| | | | | YU | 16989 | A | 31 October 1991 |
| | | | | IL | 89033 | D0 | 15 August 1989 |
| | | | | NO | 890299 | D0 | 24 January 1989 |
| | | | | BE | 1004201 | A5 | 13 October 1992 |

Form PCT/ISA/210 (patent family annex) (January 2015)

**INTERNATIONAL SEARCH REPORT**
Information on patent family members

International application No.

**PCT/CN2019/119116**

| Patent document cited in search report | Publication date (day/month/year) | Patent family member(s) | | Publication date (day/month/year) |
|---|---|---|---|---|
| | | FR | 2628109 B1 | 09 April 1993 |
| | | SE | 8900247 D0 | 24 January 1989 |
| | | CH | 681080 A5 | 15 January 1993 |
| | | GR | 890100037 A | 31 January 1990 |
| | | GB | 8901469 D0 | 15 March 1989 |
| | | ES | 2016432 A6 | 01 November 1990 |
| | | GR | 1000849 B | 17 February 1993 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ADAM A. FRIEDMAN et al.** *Nat Rev Cancer,* 2015, vol. 15 (12), 747-56
- **LIU et al.** *Am J Pathol,* 2012, vol. 180, 599-607
- **HANS CLEVERS et al.** *Cell,* 2018, vol. 172 (1-2), 373-386
- **LIU et al.** *Am J Pathol,* 2013, vol. 183 (6), 1862-1870
- **LIU et al.** *Cell Death Dis.,* 2018, vol. 9 (7), 750
- **NICK BARKER.** *Nat Cell Biol,* 2016, vol. 18 (3), 246-54
- **LIU et al.** *Nat Protoc.,* 2017, vol. 12 (2), 439-451
- **GREENWOOD et al.** *Environ Mol Mutagen,* 2004, vol. 43 (1), 36-44
- **YU et al.** *Science,* 2014, vol. 345 (6193), 216-220